# EUROPÄISCHE PATENTSCHRIFT

(11) **EP 1 187 496 B1**
(45) Veröffentlichungstag und Bekanntmachung des Hinweises auf die Patenterteilung: **29.05.2002**
(21) Anmeldenummer: 00119287.1
(22) Anmeldetag: 06.09.2000
(51) Int. Cl.: H04N 17/00

(54) **Vorrichtung zum Testen des Zustands eines Endoskops**
Device for testing the state of an endoscope
Dispositif pour tester l'état d'un endoscope

(43) Veröffentlichungstag der Anmeldung: 13.03.2002
(73) Patentinhaber: Karl Storz GmbH & Co. KG, 78532 Tuttlingen (DE)
(72) Erfinder: Dr. Fang Lei, D-78591 Durchhausen (DE); Rudischhauser Jürgen, 78532 Tuttlingen (DE); Weitzel Jörg, D-78576 Liptingen (DE); Preuss Volker, D-78462 Konstanz (DE)
(74) Vertreter: Heuckeroth, Volker, Dr.

(56) Entgegenhaltungen:
- WO-A-98/41836
- GB-A- 2 215 077
- US-A- 4 613 232
- US-A- 5 820 547

## Beschreibung

Die Erfindung betrifft eine Vorrichtung zum Testen des Zustands des optischen Systems eines Endoskops, mit einer ersten Halteeinrichtung für das Endoskop, mit einem Testbild und einer zweiten Halteeinrichtung für das Testbild.

Eine derartige Vorrichtung ist aus der US 5,820,547 bekannt.

Ein Endoskop wird insbesondere in der minival-invasiven Chirurgie als Beobachtungsinstrument verwendet, um eine durch eine kleine Inzision im Körper hindurch durchgeführte Operation von außen kontrollieren zu können.

Endoskope werden jedoch nicht nur in medizinischen Anwendungen verwendet, sondern auch in technischen Anwendungen, bspw. zur Beobachtung von mit dem bloßen Auge nicht einsehbaren Hohlräumen in Maschinen, Verbrennungsräumen von Motoren und dergleichen.

Das Hauptelement eines Endoskops ist sein optisches System. Das optische System eines Endoskopes besteht aus Stablinsen oder Glasfasern und Objektiv- und Okularlinsen. Zusätzlich kann ein Endoskop auch ein Lichtleitsystem zum Ausleuchten des Beobachtungsgebietes aufweisen. Dabei gibt es eine Vielzahl von Endoskoptypen, die sich durch ihre endoskopspezifischen Basis-Kenndaten unterscheiden. Solche endoskopspezifischen Basis-Kenndaten sind bspw. der Blickwinkel oder Blickrichtung und der Gesichtsfeldwinkel eines Endoskops. Des weiteren können sich Endoskope auch hinsichtlich des Vergrößerungsfaktors der optischen Abbildung und außerdem durch ihre geometrischen Abmessungen unterscheiden.

Hinsichtlich der Blickrichtung des Endoskops gibt es solche mit einer Geradeaus-Blickrichtung, d.h. die Blickrichtung des Endoskops bildet mit der Längsachse des Schafts des Endoskops einen Winkel von 0°. Es gibt auch Endoskope mit einer Schrägblickoptik, d.h. die Blickrichtung des Endoskops verläuft dann schräg zur Längsachse des Schafts unter einem vorgegebenen Winkel.

Das optische System eines Endoskops kann im Laufe der Zeit durch äußere Einflüsse beeinträchtigt werden, so daß sich die Qualität der Bildübertragung durch das optische System verschlechtert bzw. Abweichungen von den Sollwerten der Basis-Kenndaten auftreten. Insbesondere medizinische Endoskope werden nach jedem Gebrauch sterilisiert, wozu die Endoskope in einem Autoklaven einem unter hohem Druck stehenden heißen Dampf ausgesetzt werden. Aber auch mechanische Einflüsse wie Schläge auf den Schaft, bspw. wenn das Endoskop zu Boden fällt, können das optische System des Endoskops beschädigen.

Bei Endoskopen ist es daher erforderlich, das Endoskop im Laufe der Zeit einer Revision bzw. einer Reparatur zu unterziehen. Hierbei besteht das Problem, daß bei unsachgemäßer Durchführung der Reparatur durch nicht autorisierte Werkstätten der ursprüngliche Originalzustand bzw. Qualitätsstandard der Optik nicht erreicht wird. Für den Hersteller des Endoskops besteht daher im Rahmen des Kundenservice das Bedürfnis, den Zustand der im Bestand des Kunden befindlichen Endoskope schnell und einfach hinsichtlich der Basis-Kenndaten der Endoskope zumindest qualitativ innerhalb duldbarer Toleranzen überprüfen zu können. Dazu gehört neben der qualitativen Überprüfung der Blickrichtung und des Gesichtsfeldwinkels bspw. eine qualitative Abschätzung der Schärfe bzw. des Kontrastes der Bildübertragung in der Bildmitte und/oder im Randbereich des Bildes sowie gegebenenfalls eine Prüfung des Zustandes des Lichtleitsystems, falls das Endoskop mit einem solchen ausgestattet ist.

Aus der oben genannten US 5,820,547 ist eine tragbare und konstruktiv einfache Vorrichtung zum Testen des Zustands des optischen Systems eines Endoskops beschrieben, das eine Halteeinrichtung für das zu untersuchende Endoskop sowie ein Testbild und eine Halteeinrichtung für das Testbild umfaßt. Die Halteeinrichtung für das Endoskop besteht aus einem zylindrischen Adapter mit einer axial durchgehenden mittigen Bohrung, in die der distale Endabschnitt des Endoskops eingeschoben wird. Für Endoskope mit Schrägblickoptiken sind zwei seitliche schräg zur Längsachse des Rohrs verlaufende durchgehende Bohrungen vorgesehen, in die diese Endoskope dann entsprechend eingesteckt werden können. Das Testbild ist an einer Halteeinrichtung befestigt, die fest mit der Halteeinrichtung für das Endoskop verbunden ist. Das Testbild besteht aus einer Anordnung von Linienmustern verschiedener Linienstärke, einem Liniengitternetz sowie einer Anordnung konzentrischer Kreise.

Während diese bekannte Vorrichtung konstruktiv sehr einfach ist, hat sie jedoch den Nachteil, daß sie nicht universell an eine Vielzahl unterschiedlicher optischer Systeme von Endoskopen angepaßt ist. So ist z.B. der Abstand zwischen dem Testbild und der distalen Spitze des Endoskops fest vorgegeben. Dadurch, daß der Abstand zwischen dem Testbild und der distalen Spitze des Endoskops bei dieser bekannten Vorrichtung fest vorgegeben ist, werden bei einer Beobachtung des Testbilds durch das Endoskop in Abhängigkeit vom Gesichtsfeldwinkel jeweils unterschiedlich große Kreisausschnitte des Testbilds gesehen, was die Auswertung des Tests durch einen technisch nicht geschulten Anwender erschwert. Auch können nur Endoskopoptiken mit drei verschiedenen Blickrichtungen, die am Adapter fest vorgegeben sind, getestet werden. Die Genauigkeit des Tests ist außerdem gering. Eine Überprüfung der Funktion des Lichtleitsystems des Endoskops ist darüber hinaus nicht möglich.

Andererseits ist aus der US 5,841,525 (= WO 98/41836 A) eine Vorrichtung zum Testen des Zustands des optischen Systems eines Endoskops bekannt, die jedoch sehr aufwendig ist und zur Auswertung des Tests Peripheriegeräte wie Bildverarbeitungsgeräte und eine Rechnerstation benötigt. Diese Vorrichtung ist daher nicht transportabel und eignet sich nicht zur Durchführung von Schnelltests vor Ort bei einem Kunden.

Aus der US 4,613,232 ist ferner eine tragbare Testvorrichtung zum Testen des optischen Systems eines Endoskops bekannt, das aus einer Grundplatte und einer Schiene mit einer sich längs erstreckenden V-förmigen Nut zur Aufnahme eines Abschnitts des Endoskops besteht, das so auf der Vorrichtung angebracht wird, daß es sich parallel zu der Platte auf einem beweglichen Träger rechtwinklig zu der Nut erstreckt. Die Vorrichtung weist einen vertikalen Schaft auf, der auf der Platte montiert ist und an einem Ende in einer konischen Spitze endet, wobei der Schaft zumindest einen Verlängerungsarm trägt, der so angebracht ist, daß er in einer Ebene parallel zur Grundplatte verschwenkbar ist. Jeder Arm trägt einstellbar eine Meßscheibe, deren Höhe über der Platte oder deren Abstand von dem Schaft verändert werden kann. Die Meßscheibe weist Skalen und dergleichen auf, mit denen es möglich ist, die Blickrichtung und das Gesichtsfeld sowie den Durchmesser der Ausgangspupille, die Feldausleuchtung, die Feldtiefe, den Fokussierbereich und die Linsenvergrößerung sowie die Verzerrung und Auflösung des optischen Systems des gerade getesteten Endoskops exakt zu messen.

Der Erfindung liegt die Aufgabe zugrunde, eine Vorrichtung der eingangs genannten Art dahingehend weiterzubilden, daß sie eine schnelle qualitative Beurteilung und Überprüfung von Basis-Kenndaten bzw. der Bildqualität ohne langwierige Schulung des Anwenders ermöglicht.

Erfindungsgemäß wird diese Aufgabe hinsichtlich der eingangs genannten Vorrichtung dadurch gelöst, daß das Testbild an seinen Außensektor innen anschließend ein Muster aufweist, das eine kreisförmige Referenzlinie als Sollwert für die ideale Blickrichtung und den idealen Gesichtsfeldwinkel und ein Toleranzfeld aufweist, das aus beidseits der Referenzlinie angeordneten Ringen gebildet ist, so daß das Muster eine Überprüfung der Ist-Blickrichtung und des Ist-Gesichtsfeldwinkels innerhalb von Toleranzwerten des Sollwerts für die Blickrichtung und den Gesichtsfeldwinkel ermöglicht.

Bei dieser Ausgestaltung des Testbildes können die Blickrichtung und der Gesichtsfeldwinkel gemeinsam schnell und einfach überprüft werden, d.h. es ist möglich, schnell und einfach zu beurteilen, ob das getestete Endoskop fehlerhaft oder innerhalb der durch das Muster vorgegebenen Toleranzen in einem tauglichen Zustand ist. Mit der erfindungsgemäßen Vorrichtung können Tests von Endoskopen von Kundendienstmitarbeitern ohne langwierige vorherige Schulung vor Ort bei einem Kunden durchgeführt werden.

Durch die Ausgestaltung des Testbildes mit der Referenzlinie und dem durch die beidseits dieser Referenzlinie angeordneten Ringen gebildeten Toleranzfeld wird eine besonders einfach durchzuführende Überprüfung des Zustands der Blickrichtung und des Gesichtsfeldwinkels des Endoskops ermöglicht, weil sich die testende Person lediglich anhand einfacher geometrischer Muster orientieren muß. Die Referenzlinie entspricht dem äußeren Rand des durch ein ideales Endoskop zu beobachtenden Gesichtsfeldes.

In einer weiteren bevorzugten Ausgestaltung weist das Testbild ein Muster auf, das eine Überprüfung der Bildgüte ermöglicht.

Durch diese Ausgestaltung wird zusätzlich zu der gemeinsamen Überprüfung der Blickrichtung und des Gesichtsfeldwinkels gleichzeitig auch eine Überprüfung der Bildgüte, d.h. der Bildschärfe anhand eines geometrischen Musters ermöglicht. Das Muster zur Überprüfung der Bildgüte kann durch eine Anordnung von Linienmustern verschiedener Strichstärken auf dem Testbild gebildet sein, wobei diese auf dem Testbild so angeordnet sind, daß die Linienmuster in der Bildmitte und am Bildrand des von einem idealen Endoskop sichtbaren Bereichs des Testbildes zu erkennen sind. Bevorzugt weist das Muster eine periodische Wiederholung von Linienmustern verschiedener Strichstärken auf, so daß diese Linienmuster am Bildrand des von einem idealen Endoskop sichtbaren Bereichs der Testscheibe über den gesamten Umfang des Bildrandes zu erkennen sind.

In einer weiteren bevorzugten Ausgestaltung sind die erste Halteeinrichtung und die zweite Halteeinrichtung relativ zueinander positionierbar, so daß die relative Position des Testbilds zum Endoskop endoskopspezifisch definiert einstellbar ist.

Anstelle wie bei der aus dem Stand der Technik bekannten Vorrichtung die Halteeinrichtung für das Endoskop und die Halteeinrichtung für das Testbild unbeweglich miteinander zu verbinden, ist bei der erfindungsgemäßen Vorrichtung vorgesehen, die Halteeinrichtung für das Endoskop und die Halteeinrichtung für das Testbild relativ zueinander positionierbar, d.h. beweglich auszugestalten, so daß die relative Position des Testbilds zum Endoskop in Abhängigkeit von endoskopspezifischen Kenndaten des zu testenden Endoskops definiert einstellbar ist. Dies eröffnet vorteilhafterweise die Möglichkeit, für alle vorhandenen Typen von optischen Systemen von Endoskopen unterschiedlichster Basis-Kenndaten das Testbild so zu positionieren, daß stets der gleiche Ausschnitt des Testbilds durch die Endoskopoptik zu sehen ist, so daß Abweichungen des Endoskops vom Sollzustand leichter zu erkennen sind. Während die erste Halteeinrichtung und/oder die zweite Halteeinrichtung entsprechend den endoskopspezifischen Kenndaten bspw. anhand von aus einer Tabelle entnehmbaren Positionswerten positioniert werden, was auch ohne eine langwierige Schulung leicht erlernbar ist, ist auch die Überprüfung der Basis-Kenndaten unterschiedlicher Endoskope schnell und einfach möglich, weil stets die gleichen Merkmale des Testbildes unabhängig vom getesteten Endoskop ausgewertet werden müssen. Die erfindungsgemäße Vorrichtung ist des weiteren universell für alle vorhandenen Endoskope mit unterschiedlichsten Endoskopoptiken verwendbar und kann auch auf noch nicht vorhandene Endoskopoptiken erweitert werden.

In einer bevorzugten Ausgestaltung ist der Abstand zwischen dem Testbild und dem Endoskop einstellbar.

Hierbei ist von Vorteil, daß mit der Vorrichtung eine Vielzahl von unterschiedlichen Endoskopen getestet werden können, die sich hinsichtlich ihres vorgegebenen Gesichtsfeldwinkels unterscheiden.

In einer weiteren bevorzugten Ausgestaltung ist ein Winkel zwischen einer Normalen des Testbildes und der Längsrichtung des Endoskops einstellbar.

Hierbei ist von Vorteil, daß mit der erfindungsgemäßen Vorrichtung eine Vielzahl von unterschiedlichen Endoskopen getestet werden können, die sich hinsichtlich ihrer vorgegebenen Blickrichtung unterscheiden.

In einer weiteren bevorzugten Ausgestaltung sind den endoskopspezifischen Kenndaten Positionswerte zum Positionieren des Testbildes und/oder des Endoskops zugeordnet.

Die Verwendung von Positionswerten zum Einbringen des Endoskops in die Vorrichtung und/oder zum Positionieren des Testwertes erleichtert die Handhabung der Vorrichtung weiter. Die Positionswerte können bspw. in einer Tabelle aufgelistet und als Positionsmarken in der Vorrichtung eingetragen sein.

In einer weiteren bevorzugten Ausgestaltung weist die erste Halteeinrichtung zumindest einen auf einer sich in Längsrichtung des Endoskops erstreckenden ersten Schiene in Längsrichtung der Schiene verschiebbaren ersten Halter auf.

Diese Maßnahme hat den Vorteil, daß auch die zu testenden Endoskope selbst in der Vorrichtung in geeigneter Weise positioniert werden können. Auf diese Weise eignet sich die erfindungsgemäße Vorrichtung insbesondere auch zum Testen von Endoskopen mit unterschiedlich langen Schäften. Insbesondere in Verbindung mit der zuvor erwähnten Maßnahme, wonach der Winkel zwischen der Normalen des Testbildes und der Längsrichtung des Endoskops kenndatenspezifisch einstellbar ist, wird durch diese Maßnahme vorteilhafterweise gewährleistet, daß auch bei unterschiedlich langen Endoskopschäften stets die Normale des Testbildes mit der optischen Achse der Blickrichtung des Endoskops zusammenfällt, weil die Spitze des Endoskops stets an einem definierten Punkt in der Vorrichtung zu liegen kommt.

Dabei ist es weiterhin bevorzugt, wenn der Halter zum Befestigen des Endoskops das Endoskop in einer vorbestimmten Drehstellung bezüglich einer Längsachse des Endoskops hält.

Insbesondere bei Endoskopen mit Schrägblickoptik gewährleistet diese Maßnahme wiederum, daß die Blickrichtung des Endoskops möglichst senkrecht auf dem Testbild steht.

In einer weiteren bevorzugten Ausgestaltung weist die erste Schiene eine erste Positionierungsskala und der erste Halter einen auf der ersten Schiene axial verschiebbaren ersten Skalenreiter zur axialen Positionierung des Halters auf.

Durch die bereits zuvor erwähnte Ausgestaltung der ersten Halteeinrichtung mit einer Schiene und einem darauf verschiebbaren Halter wird vorteilhafterweise eine kontinuierlich einstellbare Positionierung des Endoskops ermöglicht, wodurch die Vorrichtung für alle vorkommenden Längen von Endoskopen geeignet ist. Durch die vorgesehene Positionierungsskala auf der Schiene und dem Skalenreiter, der auf der Schiene verschiebbar ist, kann nun eine Positionierung des jeweiligen Endoskops auf besonders einfach zu handhabende Weise anhand von tabellierten Werten vorgenommen werden. Beispielsweise können für alle vorkommenden unterschiedlichen Endoskope Positionswerte in Form von Zahlen oder Buchstaben-Zahlen-Kombinationen in einer Tabelle aufgeführt sein, wobei die Positionierungsskala dann diese Positionswerte als Markierungen aufweist. Der Skalenreiter kann zudem auch einen Nonius oder dgl. für eine Feineinstellung der Position des Endoskops aufweisen.

In entsprechender bevorzugter Ausgestaltung weist die zweite Halteeinrichtung, die das Testbild hält, einen auf einer zweiten Schiene in Längsrichtung der Schiene verschiebbaren zweiten Halter auf.

Mit diesem längsverschiebbaren Halter kann nunmehr auf einfach zu handhabende Weise der Abstand zwischen dem Testbild und der distalen Spitze des Endoskops eingestellt werden, und zwar ist der Abstand wiederum auf beliebige unterschiedliche Endoskope kontinuierlich einstellbar.

Auch hierbei ist es bevorzugt, wenn die zweite Schiene eine zweite Positionierungsskala und der zweite Halter einen auf der zweiten Schiene axial verschiebbaren Skalenreiter zur axialen Positionierung des zweiten Halters auf der zweiten Schiene aufweist.

Auf diese Weise kann der Abstand zwischen dem Testbild und der distalen Spitze des Endoskops anhand tabellierter Werte exakt eingestellt werden, indem die axiale Position des Halters des Testbildes dem tabellierten Wert entsprechend eingestellt wird.

In einer weiteren bevorzugten Ausgestaltung sind die erste Schiene und die zweite Schiene durch ein Drehgelenk miteinander verbunden.

Gegenüber der im Stand der Technik bekannten Vorrichtung, die für den Test von Endoskopen unterschiedlicher Blickrichtungen drei Bohrungen zum Einsetzen der Endoskopschäfte aufweist, hat die erfindungsgemäße Ausgestaltung der Verbindung der beiden Schienen mit einem Drehgelenk den Vorteil, daß die Möglichkeit einer kontinuierlichen Winkeleinstellung zwischen den beiden Halteeinrichtungen ermöglicht wird, wodurch Endoskope mit beliebigen Blickrichtungen getestet werden können.

Dabei ist es weiterhin bevorzugt, wenn am Drehgelenk eine umfänglich angeordnete dritte Positionierungsskala zur Einstellung des Winkels zwischen der ersten Schiene und der zweiten Schiene angeordnet ist.

Wie bei den Positionierungsskalen der ersten Halteeinrichtung und der zweiten Halteeinrichtung ist hier ebenfalls vorteilhaft, daß die für das jeweilige Endoskop erforderliche definierte Winkeleinstellung zwischen Testbild und Endoskop anhand von tabellierten Werten und somit besonders handhabungseinfach erfolgen kann.

Dabei ist es weiterhin bevorzugt, wenn das Drehgelenk eine Stellscheibe aufweist, die die dritte Positionierungsskala aufweist, und die relativ zur ersten Schiene und relativ zur zweiten Schiene drehbar ist.

Durch diese Maßnahme kann vorteilhafterweise der gesamte Umfang der Stellscheibe mit einer Positonierungsskala versehen sein, wobei durch Drehung der Stellscheibe zunächst eine Grundeinstellung und dann durch Abwinkeln der zweiten Schiene relativ zur Stellscheibe der Winkel zwischen der ersten und der zweiten Schiene eingestellt werden kann.

In einer weiteren bevorzugten Ausgestaltung weist das Testbild ein Muster auf, das eine Überprüfung der Blickrichtung, das Gesichtsfeldwinkels und/oder der Bildgüte ermöglicht.

Bei dieser Ausgestaltung des Testbildes können schnell und einfach die wesentlichen Basis-Kenndaten eines Endoskops bzw. dessen Abweichungen über vorgegebene Toleranzen hinaus ermittelt werden.

In einer weiteren bevorzugten Ausgestaltung weist die Vorrichtung weiterhin eine Lichttesteinrichtung zum Testen eines Lichtleitsystems des zu testenden Endoskops auf.

Mit dieser Maßnahme wird die Flexibilität der erfindungsgemäßen Vorrichtung weiter erhöht, weil auch ein Lichttest des Lichtleitsystems des Endoskops durchgeführt werden kann.

Damit der Lichttest mit konstruktiv einfachem Aufwand und handhabungstechnisch einfach durchgeführt werden kann, weist die Lichttesteinrichtung bevorzugt einen Photodetektor auf, der vor das lichtaustrittsseitige Ende des Endoskops bewegbar ist, und der mit einer Anzeigeeinrichtung zum Anzeigen der Lichtstärke verbunden ist.

Um den Lichttest weiter zu vereinfachen und auch für nicht technisch geschultes Personal leicht auswertbar zu gestalten, weist die Anzeigeeinrichtung weiterhin eine Kalibrierungseinrichtung, bspw. ein Potentiometer, auf, mittels der die Anzeigeeinrichtung auf einen Referenzwert einstellbar ist.

Mit derselben Lichtquelle, die für den Lichttest am Endoskop verwendet wird, kann zunächst der Photodetektor direkt bestrahlt werden, d.h. ohne daß das Licht durch das Endoskop geleitet wird, und die Anzeigeeinrichtung kann auf einen vorbestimmten Referenzwert kalibriert werden. Anschließend wird dann mit derselben Lichtquelle das Licht durch das Lichtleitsystem des Endoskops auf den Photodetektor gerichtet, und die den Test durchführende Person kann dann auf der Anzeigeeinrichtung den Ist-Wert der Lichtstärke ablesen und mit einem Sollwert in einer Tabelle vergleichen.

Um die Referenzmessung weiter zu vereinfachen, weist die Lichttesteinrichtung bevorzugt einen Lichtkabelanschluß zum Anschließen eines Lichtkabels auf, wobei der Photodetektor in eine Stellung bringbar ist, in der er dem Lichtkabelanschluß benachbart angeordnet ist.

In einer weiteren bevorzugten Ausgestaltung ist der Photodetektor an einer dritten Halteeinrichtung befestigt, die einen an einem mit der zweiten Schiene verbundenen Arm verschwenkbar angeordnet Halter aufweist.

Durch diese Maßnahme ist die Lichttesteinrichtung vorteilhafterweise in die zweite Halteeinrichtung für das Testbild integriert, und läßt sich der Photodetektor vorteilhafterweise einfach vor das distale Ende des zu testenden Endoskops und vor den Lichtkabelanschluß für die Referenzmessung bewegen. Durch die Anbringung des Halters an der zweiten Schiene, die relativ zur ersten Schiene entsprechend der Blickrichtung des Endoskops abgewinkelt wird, trifft auch das Licht aus dem Endoskop senkrecht auf den Photodetektor.

Weitere Merkmale und Vorteile ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine schematische Gesamtansicht einer Vorrichtung zum Testen des Zustands des optischen Systems eines Endoskops; und
- Fig. 2: ein in der Vorrichtung in Fig. 1 verwendetes Testbild in gegenüber Fig. 1 stark vergrößertem Maßstab.

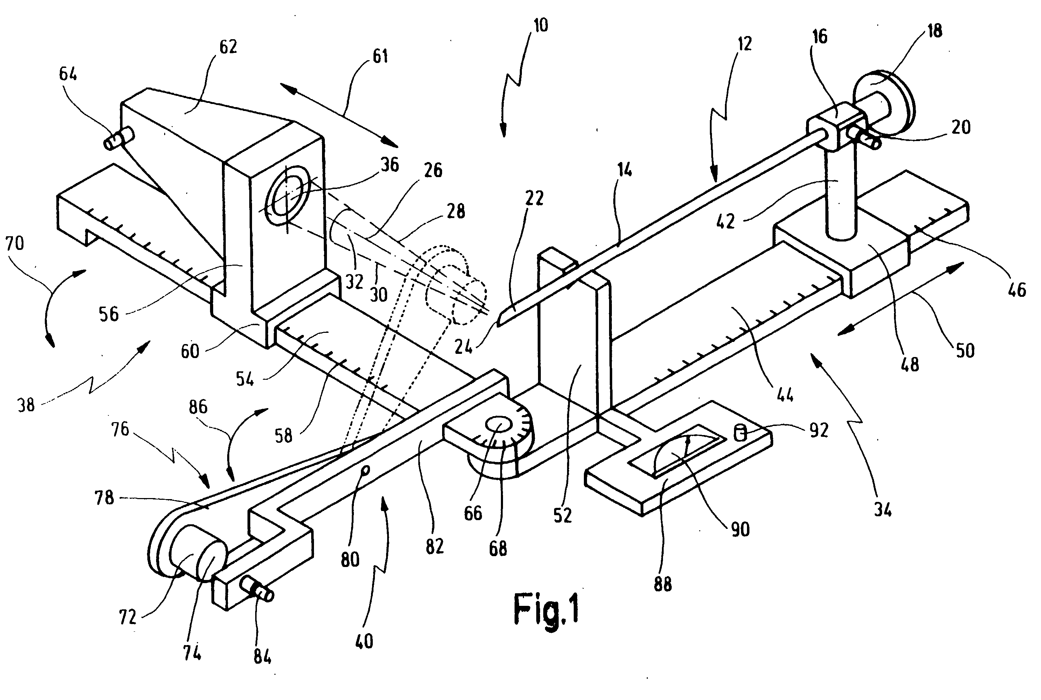

In Fig. 1 ist eine mit dem allgemeinen Bezugszeichen 10 versehene Vorrichtung zum Testen des Zustands des optischen Systems eines Endoskops 12 dargestellt. Die Vorrichtung 10 ist insgesamt transportabel und kann auf einem Tisch aufgestellt werden.

Mit der Vorrichtung 10 kann der Zustand des optischen Systems des Endoskops 12 in einer "Gut-Schlecht"-Abschätzung getestet werden, d.h. ob der Ist-Zustand des Endoskops 12 die bei seiner Herstellung vorgegebenen Sollwerte innerhalb vorbestimmter Toleranzen noch erfüllt oder die Ist-Werte von den Sollwerten so weit abweichen, daß das Endoskop 12 nicht mehr den Qualitätsanforderungen genügt. Überprüfbar sind die Blickrichtung und der Gesichtsfeldwinkel des optischen Systems des Endoskops sowie die Bildgüte, d.h. die Schärfe bzw. der Kontrast des Bildes, wobei die Bildgüte in der Bildmitte und in Außenbereichen des Bildes möglich ist. Des weiteren kann das Lichtleitsystem des Endoskops 12 getestet werden.

Bevor auf die Vorrichtung 10 näher eingegangen wird, wird zunächst kurz das Endoskop 12 beschrieben. Das Endoskop 12 weist einen langerstreckten starren Schaft 14 auf, in dem das optische System in Form eines Relais-Stablinsensystems oder eines geordneten Glasfaserbündels angeordnet ist. Am proximalen Ende des Schafts 14 weist das Endoskop 12 ein Okulargehäuse 16 auf, an das sich eine Okularmuschel 18 anschließt. Das optische System des Endoskops 12 umfaßt auch die in dem Okulargehäuse 16 bzw. der Okularmuschel 18 vorhandenen Linsen sowie etwaige Objektivlinsen am distalen Ende des Schafts 14.

Am Okulargehäuse 16 ist ferner ein Lichtkabelanschluß 20 zum Anschließen eines nicht dargestellten Lichtkabels einer nicht dargestellten Lichtquelle angeordnet. Entsprechend weist das Endoskop 12 ein sich durch den Schaft 14 erstreckendes Lichtleitsystem in Form von ungeordneten optischen Fasern auf, mit dem Licht von einer externen Lichtquelle über den Lichtkabelanschluß 20 bis zu einer distalen Spitze bzw. einem distalen Ende 22 des Schafts 14 geleitet wird, von wo es dann aus dem Schaft 14 austritt.

Das distale Ende 22 des Schafts 14 weist in dem gezeigten Ausführungsbeispiel des Endoskops 14 eine abgeschrägte Stirnfläche 24 auf, die gleichzeitig das lichteintritts- bzw. lichtsaustrittsseitige Ende des Endoskops 12 bildet. Die abgeschrägte Stirnfläche 24 deutet in Fig. 1 an, daß das Endoskop 12 eine Schrägblickoptik aufweist, d.h. eine mit einer strichpunktierten Linie angedeutete Blickrichtung 26 verläuft schräg zur Längsrichtung des Endoskops 12, die durch die Längsrichtung des Schafts 14 definiert wird. Im dargestellten Ausführungsbeispiel weist das Endoskop 12 eine 90°-Optik auf, d.h. die Blickrichtung 26 verläuft senkrecht zur Längsachse des Endoskops 12. Auch das durch das lichtzuführende System des Endoskops 12 aus der distalen Stirnfläche 24 austretende Licht wird mit seiner Hauptrichtung in Richtung der Blickrichtung 26 abgestrahlt.

Die Blickrichtung 26 in Fig. 1 stellt nur die Symmetrieachse bzw. optische Achse des Gesichtsfeldes des optischen Systems des Endoskops 12 dar. Mit unterbrochenen Linien 28 und 30 sind äußere Grenzen des Gesichtsfeldes des optischen Systems des Endoskops 12 angedeutet. Das Gesichtsfeld läßt sich durch einen Kegelmantel beschreiben, dessen Kegelwinkel der Gesichtsfeldwinkel 32 des optischen Systems des Endoskops 12 ist, der neben der Blickrichtung 26 relativ zur Längsrichtung des Endoskops 12 ein weiterer Basis-Kennwert des Endoskops 12 ist.

Im folgenden wird nun die Vorrichtung 10 näher beschrieben.

Die Vorrichtung 10 weist als Hauptbaugruppen eine erste Halteeinrichtung 34 für das Endoskop 12, ein Testbild 36 sowie eine zweite Halteeinrichtung 38 für das Testbild 36 auf. Des weiteren weist die Vorrichtung 10 eine Lichttesteinrichtung 40 zum Testen des Lichtleitsystems des Endoskops 12 auf.

Die erste Halteeinrichtung 34 weist einen ersten Halter 42 auf. Der Halter 42 ist auf einer Schiene 44 in Längsrichtung der Schiene 44 verschiebbar angeordnet. Auf der Schiene 44 ist eine Positionierungsskala 46 vorgesehen, die in Fig. 1 nur schematisch und grob dargestellt ist. Der Halter 42 weist an seinem Fuß einen auf der Schiene 44 axial verschiebbar angeordneten Skalenreiter 48 auf. Der Skalenreiter 48 ist gemäß einem Doppelpfeil 50 entlang der Schiene 44 verschiebbar.

Die Positionierungsskala 46 kann auf verschiedene Weise gebildet sein. Beispielsweise kann die Positionierungsskala 46 eine Längenskala mit Millimetereinteilung sein oder eine Skala, die an jedem Skalenstrich eine Zahlen- oder Buchstaben-Zahlen-Kombination anzeigt. Der Skalenreiter 48 kann mit einer nicht dargestellten entsprechenden Markierung in Form eines Striches, einem Nonius, ggf. mit einer Lupe, ausgestattet sein, so daß der Skalenreiter 48 exakt an einer definierten Stelle auf der Schiene 44 positioniert werden kann. Die Positionierung des Skalenreiters 48 auf der Schiene 44 anhand der Positionierungsskala 46 erfolgt dabei anhand tabellierter Werte, d.h. für jedes Endoskop bzw. jeden Endoskoptyp einer bestimmten Artikelnummer ist in einer der Vorrichtung 10 beigefügten Tabelle zu entnehmen, an welcher Stelle der Skalenreiter 48 und damit der Halter 42 und damit wiederum das Endoskop 12 auf der Schiene 44 für den durchzuführenden Test zu positionieren ist.

Der Halter 42 weist zum Befestigen des Endoskops 12 an dem Halter 42 eine nicht näher dargestellte Kupplung auf, mit der das Endoskop 12 in einer vorbestimmten Drehstellung bezüglich seiner Längsachse in der Halteeinrichtung 34 gehalten ist. Da das Endoskop 12 aufgrund seiner Schrägblickoptik bezüglich der Längsrichtung des Endoskops 12 nicht rotationssymmetrisch ist, ist eine Befestigung des Endoskops 12 in einer vorbestimmten Drehstellung bezüglich seiner Längsachse für die Durchführung des Tests erforderlich.

Die Kupplung zum Befestigen des Endoskops 12 an dem Halter 42 kann eine Konus-Kupplung sein, die automatisch nur in einer bestimmten Drehstellung des Endoskops 12 bezüglich seiner Längsachse ermöglicht wird, und die von distal gegen einen entsprechenden Gegenkonus am Okulargehäuse 16 verriegelt wird.

Die erste Halteeinrichtung 34 weist weiterhin einen weiteren Halter 52 auf, der lediglich der Abstützung des distalen Bereichs des Schafts 14 des Endoskops 12 dient. Der weitere Halter 52 kann ebenfalls auf der Schiene 44 in deren Längsrichtung verschiebbar sein. Der weitere Halter 52 dient jedoch nicht zur Positionierung des Endoskops 12. Zur Positionierung des Endoskops 12 dient wie zuvor beschrieben allein der Halter 42 der ersten Halteeinrichtung 34.

Die zweite Halteeinrichtung 38 weist einen auf einer zweiten Schiene 54 in Längsrichtung der Schiene 54 verschiebbaren zweiten Halter 56 auf. Der zweite Halter 56 kann das Testbild 36, das in Form einer Karte oder Platte ausgebildet ist, fest integriert enthalten, oder der Halter 56 kann einen Schacht zum Einstecken des Testbildes 36 aufweisen, so daß das Testbild 36 von dem Halter 56 abgenommen werden kann.

Die zweite Schiene 54 weist eine Positionierungsskala 58 auf, wobei der zweite Halter 56 einen auf der zweiten Schiene 54 axial verschiebbaren Skalenreiter 60 aufweist, mit dem der zweite Halter 56 auf der zweiten Schiene 54 axial positioniert werden kann. Der Skalenreiter 60 und damit der zweite Halter 56 und damit das Testbild 36 können somit entlang der zweiten Schiene 54 gemäß einem Doppelpfeil 61 verschoben und an entsprechender Stelle positioniert werden.

Mit Hilfe der Positionierungsskala 58 kann somit der Abstand zwischen dem Testbild 36 und dem Endoskop 12, genauer gesagt dem distalen Ende 22 bzw. der Stirnfläche 24, endoskopspezifisch eingestellt werden. Die Einteilung der Positionierungsskala 58 und die Ausgestaltung des Skalenreiters 60 kann dabei der Einteilung der Positionierungsskala 46 und der Ausgestaltung des Skalenreiters 48 entsprechen. Die Positionierungsskala 58 unterscheidet sich jedoch von der Positionierungsskala 46 hinsichtlich der angegebenen Positionswerte, um Verwechslungen zwischen der Positionierungsskala 46 und der Positionierungsskala 58 beim Positionieren des Endoskops 12 einerseits und dem Positionieren der Testscheibe 36 andererseits zu vermeiden.

Wiederum ist für jedes zu testende Endoskop 12 bzw. für jeden Endoskoptyp der der Vorrichtung 10 beigefügten Tabelle ein Positionswert zu entnehmen, auf den der Skalenreiter 60 auf der Positionierungsskala 58 eingestellt werden kann.

Der Halter 56 für das Testbild 36 weist weiterhin ein Gehäuse 62 auf, an dem ein Lichtkabelanschluß 64 vorgesehen ist, an den ein nicht dargestelltes Lichtkabel angeschlossen werden kann, um das Testbild 36 von seiner Rückseite aus zu beleuchten. Das Testbild 36 ist dazu transparent, in der Art einer Mattscheibe, ausgebildet.

Bei der Vorrichtung 10 ist des weiteren ein Winkel zwischen einer Normalen des Testbildes 36, d.h. der auf der Fläche des Testbildes stehenden Senkrechten, und der Längsrichtung des Endoskops 12 einstellbar.

Dazu sind die erste Halteeinrichtung 34 und die zweite Halteeinrichtung 38, d.h. genauer gesagt die erste Schiene 44 und die zweite Schiene 54 miteinander über ein Drehgelenk 66 verbunden.

Am Drehgelenk 66 ist eine nicht dargestellte Stellscheibe mit einer umfänglich angeordneten Positionierungsskala 68 zur Einstellung des Winkels zwischen der ersten Schiene 44 und der zweiten Schiene 54 angeordnet.

Die Markierungen können Gradangaben aufweisen, oder wiederum Zahlen- oder Buchstaben-Zahlen-Kombinationen. Jedem zu testenden Endoskop bzw. Endoskoptyp ist in der bereits genannten Tabelle der entsprechende Positionierungswinkel bzw. Positionswert der Positionierungsskala 68 zugeordnet, so daß der vorgegebene Tabellenwert an der Stellscheibe bzw. am Drehgelenk 66 eingestellt werden kann.

Während die Einstellung des Abstands der Testscheibe 36 vom Endoskop 12 den unterschiedlichen Gesichtsfeldwinkeln unterschiedlicher Endoskope bzw. Endoskoptypen Rechnung trägt, trägt die Einstellung des Winkels zwischen der Normalen des Testbilds 36 und der Längsrichtung des Endoskops 12 den unterschiedlichen Blickrichtungen verschiedener Endoskope bzw. verschiedener Endoskoptypen Rechnung.

Die zuvor genannte Stellscheibe, die am Drehgelenk 66 angeordnet ist, kann weiterhin relativ zur ersten Schiene 44 und zur zweiten Schiene 54 drehbar sein, so daß der volle Umfang der Stellscheibe für die Positionierungsskala 68 genutzt werden kann, indem die Stellscheibe bspw. in zwei oder mehr Grundpositionen voreinstellbar ist, und anschließend die zweite Schiene 54 dann relativ zur Stellscheibe in den vorgegebenen Winkel gedreht werden kann. Die zweite Schiene 54 läßt sich bevorzugt über einen Winkelbereich von 0° (die zweite Schiene 54 steht in geradliniger Verlängerung zur ersten Schiene 44) bis zu einem Winkel von zumindest 120° oder darüber abwinkeln, so daß auch Endoskope mit Rückwärtsblickrichtung getestet werden können. Die Winkelverstellung der zweiten Schiene 54 relativ zur ersten Schiene 44 ist mit einem Doppelpfeil 70 angedeutet.

Insgesamt sind somit die erste Halteeinrichtung 34 und die zweite Halteeinrichtung 38 relativ zueinander so positionierbar, daß die relative Position des Testbilds 36 zum Endoskop 12 in Abhängigkeit von endoskopspezifischen Kenndaten des zu testenden Endoskops 12 definiert einstellbar ist. Die endoskopspezifischen Kenndaten sind dabei bevorzugt in der bereits genannten der Vorrichtung 10 beigefügten Tabelle enthalten, in der alle Endoskope bzw. Endoskoptypen des Herstellers mit den entsprechenden Positionswerten für die Positionierungsskalen 46 und 58 bzw. 68 aufgeführt sind.

Die Halteeinrichtung 38, die Halteeinrichtung 34 oder das Drehgelenk 66 sind weiterhin bevorzugt in den tabellarisch vorgegebenen Positionen rastbar, um die Gefahr einer unerwünschten Verstellung der Position während der Durchführung des Tests zu vermeiden oder zu verringern oder das Auffinden der exakten Position zu erleichtern.

Die zuvor erwähnte Lichttesteinrichtung 40 zum Testen des Lichtleitsystems des Endoskops 12 weist einen Photodetektor 72 auf, der eine lichtempfindliche Stirnseite 74 aufweist.

Der Photodetektor 72 ist an einer dritten Halteeinrichtung 76 befestigt, die einen Halter 78 aufweist, an dem der Photodetektor 72 befestigt ist. Der Halter 78 ist über ein Drehgelenk 80 verschwenkbar an einem Arm 82 befestigt, der wiederum an der zweiten Schiene 54 befestigt ist.

Am freien Ende des Arms 82 ist weiterhin ein Lichtkabelanschluß 84 angeordnet, an den ein nicht dargestelltes Lichtkabel angeschlossen werden kann, das mit einer nicht dargestellten externen Lichtquelle verbunden ist. Der Photodetektor 72 kann durch Verschwenken des Halters 78 einerseits in eine Position gebracht werden, in der er dem Lichtkabelanschluß 84 benachbart ist, wie in Fig. 1 mit durchgezogenen Linien dargestellt ist, und er kann durch Verschwenken des Halters 78 in eine Position gebracht werden, in der er vor das lichtaustrittsseitige Ende, d.h. vor die Stirnfläche 24 des Endoskops 12 gebracht werden kann, wie in Fig. 1 mit unterbrochenen Linien dargestellt ist. Die Verschwenkbarkeit des Halters 78 ist mit einem Doppelpfeil 86 veranschaulicht.

Die Lichttesteinrichtung 40 weist weiterhin eine Anzeigeeinrichtung 88 zum Anzeigen der durch den Photodetektor 72 gemessenen Lichtstärke auf. Die Anzeigeeinrichtung 88 ist mit dem Photodetektor 72 über eine entsprechende nicht dargestellte elektrische Leitung verbunden.

Die Anzeigeeinrichtung 88 kann eine Analoganzeige 90 oder eine Digitalanzeige aufweisen.

Die Anzeigeeinrichtung 88 weist weiterhin eine Kalibrierungseinrichtung 92 auf, mittel der die Anzeigeeinrichtung 88 auf einen Referenzwert einstellbar ist, wie hiernach noch beschrieben wird.

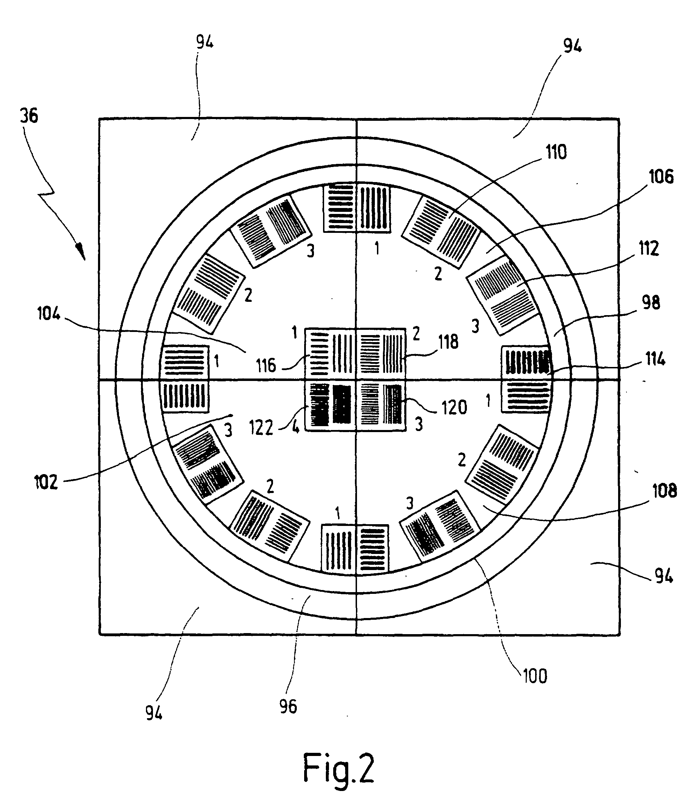

In Fig. 2 ist das Testbild 36 in Alleinstellung dargestellt.

Das Testbild 36 ist mit einem Muster versehen, das eine Überprüfung im Sinne einer "Gut-Schlecht"-Abschätzung der Basis-Kenndaten des zu testenden Endoskops 12 innerhalb vorgegebener Toleranzwerte ermöglicht. Das Testbild 36 ermöglicht im vorliegenden Fall die Überprüfung der Blickrichtung 26, des Gesichtsfeldwinkels 32 sowie der Bildgüte, d.h. der Schärfe und des Kontrasts der Bildübertragung in der Bildmitte und im Außenbereich des Endoskopbildes.

Von außen nach innen weist das Testbild 36 einen vollumfänglich einheitlichen Außensektor 94 auf, der mit einer Farbe, bspw. rot, vollständig ausgefüllt ist. An den Außensektor 94 schließen sich zwei Ringe 96 und 98 an, die durch eine kreisförmige Referenzlinie 100 voneinander getrennt sind. Beide Ringe 96 und 98 sind über ihren gesamten Umfang in der gleichen Farbe, bspw. grün, zur Unterscheidung von dem Außensektor 94 ausgefüllt. Die Referenzlinie 100 stellt den Sollwert für die Blickrichtung 26 und den Gesichtsfeldwinkel 32 für den Idealzustand des Endoskops 12 dar. Die Ringe 96, 98 beidseits der Referenzlinie stellen Toleranzfelder dar, innerhalb derer die Ist-Blickrichtung und der Ist-Gesichtsfeldwinkel abweichen dürfen.

Der gesamte Bereich des Testbilds 36 innerhalb des Rings 98 ist mit weißem Hintergrund ausgeführt, auf dem in vier Quadranten 102-108 in deren Außenbereich sich von Quadrant zu Quadrant wiederholende Linienfelder 110, 112 und 114 angeordnet sind. Die Linienfelder 110, 112, 114 sind mit Kennzahlen 1, 2 und 3 für unterschiedliche Linienstärken gekennzeichnet.

Im Zentrum des Testbildes 36 sind vier Linienfelder 116-122 angeordnet, die mit den Stärkenkennzahlen 1 bis 4 bezeichnet sind und deren Linienstärke vom Linienfeld 116 zum Linienfeld 122 abnimmt.

Im folgenden wird nun ein Verfahren zum Testen des Endoskops 12 mittels der Vorrichtung 10 beschrieben.

Bevor das Endoskop 12 in die Vorrichtung 10 eingebracht wird, werden die Halter 42 und 56 auf den Schienen 44 bzw. 54 positioniert und außerdem der Winkel zwischen der Schiene 54 und der Schiene 44 eingestellt. Die Positionswerte für die Positionierung des Skalenreiters 48 auf der Positionierungsskala 46, des Skalenreiters 60 auf der Positionierungsskala 58 sowie der Positionswert für die Positionierungsskala 68 am Drehgelenk 66 sind für das zu testende Endoskop 12 in der genannten Tabelle aufgeführt und werden entsprechend den Tabellenwerten eingestellt.

Hierbei sei erwähnt, daß der Skalenreiter 60 vorteilhafterweise von dem Halter 56 getrennt ausgeführt ist, und als Anschlag für den Halter 56 dient, der dann mit einem eigenen Reiter auf der Schiene 54 verschiebbar angeordnet ist. Dadurch kann der Halter 56 mit dem Testbild 36 bei der Durchführung des Lichttests mit der Lichttesteinrichtung 40 beiseite geschoben werden, ohne den Positionswert für den späteren Test mit dem Testbild 36 zu verlieren.

Nachdem alle Positionierungen anhand der vorgegebenen Tabellenwerte definiert vorgenommen wurden, wird das Endoskop 12 an dem Halter 42 und an dem Halter 52 befestigt. Wie bereits erwähnt, legt der Halter 42 nicht nur die axiale Position des Endoskops 12 auf der ersten Schiene 44 sondern auch seine Drehstellung bezüglich seiner Längsachse fest, wie in Fig. 1 dargestellt ist. Durch die kenndatenspezifische Positionierung des Endoskops 12 auf der ersten Schiene 44 kommt das distale Ende 22 bzw. die Stirnfläche 24 über dem Drehgelenk 66 zu liegen. Die Stirnfläche 24 zeigt dabei in Richtung des Testbildes 36. Die kenndatenspezifische Positionierung des Testbilds 36 dient der Einstellung des für den Test erforderlichen richtigen Abstands des Testbilds 36 zur Stirnfläche 24 des Endoskops 12.

Wird das Endoskop 12 nun am Halter 42 befestigt, sollte bei einem ordnungsgemäßen Endoskop dieses Typs die Blickrichtung 26 senkrecht auf der Mitte des Testbildes 36 stehen.

Nach dem Einspannen des Endoskops 12 in die erste Halteeinrichtung 34 kann nunmehr der Test unmittelbar beginnen. Zur Durchführung des Tests kann dazu mit dem bloßen Auge durch das Okular 18 geblickt werden, um das Testbild 36 unmittelbar durch das Endoskop 12 zu beobachten, oder an das Okular 18 kann eine Videokamera, falls vorhanden, angeschlossen werden, und das durch das Endoskop 12 übertragene Bild kann dann auf einen Monitor zur Darstellung gebracht werden.

Wenn bei der Beobachtung des Testbilds 36 das durch den Ring 98 und/oder den Ring 96 gebildete Toleranzbild am gesamten Bildrand zu erkennen ist, ist die Kombinationsprüfung des Gesichtsfeldwinkels 32 und der Blickrichtung 26 des Endoskops 12 innerhalb der gemeinsamen Toleranz in Ordnung, d.h. das Endoskop 12 ist hinsichtlich der Kombination dieser Basis-Kenndaten in einem ordnungsgemäßen Zustand. Ist am Bildrand einer der Quadranten 102-108 des Innenbereiches und/oder der Außensektor 94 zu erkennen, so ist die Kombination von Blickrichtung 26 und Gesichtsfeldwinkel 32 des Endoskops 12 außerhalb der gemeinsamen Toleranz, also fehlerhaft.

Mit Hilfe der Linienfelder 116-122 kann des weiteren die Bildgüte in der Mitte des Bildes getestet werden. Dabei kann das jeweilige Linienmuster als aufgelöst betrachtet werden, wenn alle Linien des jeweiligen Linienfeldes 116-122 einzeln zu erkennen sind.

Hierbei kann notiert werden, bis zu welcher Stärke 1 bis 4 die Linien der Linienfelder 116-122 noch aufgelöst werden können.

Bezüglich der Beurteilung der Bildgüte kann anschließend noch ein Vergleichstest mit einem neuen Endoskop des gleichen Typs durchgeführt werden, wodurch sich Aufschlüsse über die Veränderung der Bildgüte des Endoskops mit dem Alter des Endoskops ergeben.

Ebenso kann wie vorstehend beschrieben die Bildgüte am Bildrand anhand der Linienfelder 110-114 abgeschätzt werden, wobei in den einzelnen Quadranten 102-106 die Bildgüte von Quadrant zu Quadrant gleich sein sollte. Auch hier kann wiederum ein Vergleichstest mit einem neuen Endoskop des gleichen Typs durchgeführt werden.

Als nächstes kann nun mit der Lichttesteinrichtung 40 das Lichtleitsystem des Endoskops 12 durchgeführt werden.

Zunächst wird der Halter 78 mit dem Photodetektor 72 in die in Fig. 1 durchgezogene Linie dargestellte Stellung verschwenkt. An den Lichtkabelanschluß 84 wird ein Lichtkabel einer Lichtquelle zur Durchführung der Referenzaufnahme angeschlossen. Nach Einschalten der Lichtquelle ist an der Anzeigeeinrichtung 88 ein Zeigerausschlag oder eine Zahl sichtbar. Zur Kalibrierung der Anzeigeeinrichtung 88 wird die Kalibrierungseinrichtung 92, bspw. in Form eines Potentiometers, betätigt, und die Anzeige 90 ggf. durch Verstellen der Leistung der Lichtquelle so eingestellt, daß auf der Anzeige 90 ein Referenzwert, bspw. 100, eingestellt ist.

Das Lichtkabel wird anschließend von dem Lichtkabelanschluß 84 abgenommen und an den Lichtkabelanschluß 20 des Endoskops 12 angeschlossen.

Der Halter 78 mit dem Photodetektor 72 wird dann in die in Fig. 1 mit unterbrochenen Linien dargestellte Stellung verschwenkt. Der Photodetektor 72 mißt nunmehr die Lichtstärke des Lichtleitsystems des Endoskops 12. Aus der Anzeigeeinrichtung 90 ergibt sich nunmehr ein von dem voreingestellten Referenzwert möglicherweise abweichender Meßwert, der von dem Benutzer der Vorrichtung 10 notiert wird. Für den Lichttest ist wiederum für jedes Endoskop bzw. jeden Endoskoptyp in der Tabelle ein Sollwert entnehmbar. Ist der aktuelle Meßwert an der Anzeige 90 größer als der Tabellenwert, ist das Lichtleitsystem des Endoskops 12 in einem ordnungsgemäßen Zustand.

## Patentansprüche

1. Vorrichtung zum Testen des Zustands des optischen Systems eines Endoskops (12), mit einer ersten Halteeinrichtung (34) für das Endoskop (12), mit einem Testbild (36) und einer zweiten Halteeinrichtung (38) für das Testbild (36), **dadurch gekennzeichnet, daß** das Testbild (36) an seinen außensektor (94) innen anschließend ein Muster aufweist, das eine kreisförmige Referenzlinie (100) als Sollwert für die ideale Blickrichtung und den idealen Gesichtsfeldwinkel und ein Toleranzfeld aufweist, das aus beidseits der Referenzlinie (100) angeordneten Ringen (96, 98) gebildet ist, so daß das Muster eine Überprüfung der Ist-Blickrichtung und des Ist-Gesichtsfeldwinkels innerhalb von durch das Toleranzfeld vorgegebenen Toleranzwerten des Sollwertes für die Blickrichtung und den Gesichtsfeldwinkel ermöglicht.

2. Vorrichtung nach Anspruch 1, **dadurch gekennzeichnet, daß** das Testbild ein Muster aufweist, das eine Überprüfung der Bildgüte ermöglicht.

3. Vorrichtung nach Anspruch 1 oder 2, **dadurch gekennzeichnet, daß** die erste Halteeinrichtung (34) und die zweite Halteeinrichtung (38) relativ zueinander positionierbar sind, so daß die relative Position des Testbilds (36) zum Endoskop (12) endoskopspezifisch definiert einstellbar ist.

4. Vorrichtung nach Anspruch 3, **dadurch gekennzeichnet, daß** der Abstand zwischen dem Testbild (36) und dem Endoskop (12) einstellbar ist.

5. Vorrichtung nach Anspruch 3 oder 4, **dadurch gekennzeichnet, daß** ein Winkel zwischen einer Normalen des Testbildes (36) und der Längsrichtung des Endoskops (12) einstellbar ist.

6. Vorrichtung nach einem der Ansprüche 3 bis 5, **dadurch gekennzeichnet, daß** endoskopspezifischen Kenndaten des Endoskops (12) Positionswerte zum Positionieren des Testbildes (36) und/oder des Endoskops (12) zugeordnet sind.

7. Vorrichtung nach einem der Ansprüche 1 bis 6, **dadurch gekennzeichnet, daß** die erste Halteeinrichtung (34) zumindest einen auf einer sich in Längsrichtung des Endoskops (12) erstreckenden ersten Schiene (44) in Längsrichtung der Schiene (44) verschiebbaren ersten Halter (42) aufweist.

8. Vorrichtung nach Anspruch 7, **dadurch gekennzeichnet, daß** der Halter (42) zum Befestigen des Endoskops (12) das Endoskop (12) in einer vorbestimmten Drehstellung bezüglich einer Längsachse des Endoskops (12) hält.

9. Vorrichtung nach Anspruch 5 oder 8, **dadurch gekennzeichnet, daß** die erste Schiene (44) eine erste Positionierungsskala (46) und der erste Halter (42) einen auf der ersten Schiene (44) axial verschiebbaren ersten Skalenreiter (48) zur axialen Positionierung des Halters (42) aufweist.

10. Vorrichtung nach einem der Ansprüche 1 bis 9, **dadurch gekennzeichnet, daß** die zweite Halteeinrichtung (38) einen auf einer zweiten Schiene (54) in Längsrichtung der Schiene (54) verschiebbaren zweiten Halter (56) aufweist.

11. Vorrichtung nach Anspruch 10, **dadurch gekennzeichnet, daß** die zweite Schiene (54) eine zweite Positionierungsskala (58) und der zweite Halter (56) einen auf der zweiten Schiene (54) axial verschiebbaren Skalenreiter (60) zur axialen Positionierung des zweiten Halters (56) auf der zweiten Schiene (54) aufweist.

12. Vorrichtung nach einem der Ansprüche 10 oder 11, **dadurch gekennzeichnet, daß** die erste Schiene (44) und die zweite Schiene (54) durch ein Drehgelenk (66) miteinander verbunden sind.

13. Vorrichtung nach Anspruch 12, **dadurch gekennzeichnet, daß** am Drehgelenk (66) eine umfänglich angeordnete dritte Positionierungsskala (68) zur Einstellung des Winkels zwischen der ersten Schiene (44) und der zweiten Schiene (54) angeordnet ist.

14. Vorrichtung nach Anspruch 13, **dadurch gekennzeichnet, daß** am Drehgelenk (66) eine Stellscheibe angeordnet ist, die die dritte Positionierungsskala (68) aufweist, und die relativ zur ersten Schiene (44) und relativ zur zweiten Schiene (54) drehbar ist.

15. Vorrichtung nach einem der Ansprüche 1 bis 14, **dadurch gekennzeichnet, daß** sie weiterhin eine Lichttesteinrichtung (40) zum Testen eines Lichtleitsystems des zu testenden Endoskops (12) aufweist.

16. Vorrichtung nach Anspruch 15, **dadurch gekennzeichnet, daß** die Lichttesteinrichtung (40) einen Photodetektor (72) aufweist, der vor das lichtaustrittsseitige Ende des Endoskops (12) bewegbar ist, und der mit einer Anzeigeeinrichtung (88) zum Anzeigen der Lichtstärke verbunden ist.

17. Vorrichtung nach Anspruch 16, **dadurch gekennzeichnet, daß** die Anzeigeeinrichtung (88) eine Kalibrierungseinrichtung (92), bspw. ein Potentiometer, aufweist, mittels der die Anzeiggeeinrichtung (88) auf einen Referenzwert einstellbar ist.

18. Vorrichtung nach Anspruch 16 oder 17, **dadurch gekennzeichnet, daß** die Lichttesteinrichtung (40) einen Lichtkabelanschluß (84) zum Anschließen eines Lichtkabels aufweist, wobei der Photodetektor (72) in eine Stellung bringbar ist, in der er dem Lichtkabelanschluß (84) benachbart angeordnet ist.

19. Vorrichtung nach einem der Ansprüche 16 bis 18, soweit diese auf Anspruch 10 oder 11 rückbezogen sind, **dadurch gekennzeichnet, daß** der Photodetektor (72) an einer dritten Halteeinrichtung (76) befestigt ist, die einen an einem mit der zweiten Schiene (54) verbundenen Arm (82) verschwenkbar angeordneten Halter (78) aufweist.

## Claims

1. An apparatus for testing the state of the optical system of an endoscope (12), comprising a first supporting device (34) for the endoscope (12), a test pattern (36) and a second supporting device (38) for the test pattern (36), **characterized in that** the test pattern (36) comprises a pattern adjacent to the inside of its outer sector (94), which pattern comprises a circular reference line (100) as nominal value for the ideal viewing direction and for the ideal field of view angle, and a tolerance field, which is formed by rings (96, 98) that are arranged on both sides of the reference line (100), so that the pattern allows a check of the actual viewing direction and of the actual field of view angle within tolerance values of the nominal value for the viewing direction and the field of view angle predetermined by the tolerance field.

2. The apparatus of claim 1, **characterized in that** the test pattern comprises a pattern, which allows a check of the image quality.

3. The apparatus of claim 1 or 2, **characterized in that** the first supporting device (34) and the second supporting device (38) can be positioned relative to each other, so that the relative position of the test pattern (36) with respect to the endoscope (12) is endoscope-specifically adjustable.

4. The apparatus of claim 3, **characterized in that** the distance between the test pattern (36) and the endoscope (12) is adjustable.

5. The apparatus of claim 3 or 4, **characterized in that** an angle between a normal line of the test pattern (36) and the longitudinal direction of the endoscope (12) can be adjusted.

6. The apparatus of anyone of claims 3 through 5, **characterized in that** positional values for positioning the test pattern (36) and/or the endoscope (12) are allocated to endoscope-specific data of the endoscope (12).

7. The apparatus of anyone of claims 1 through 6, **characterized in that** the first supporting device (34) comprises at least one first support (42) displaceable on a first rail (44) in longitudinal direction, the rail extending in longitudinal direction of the endoscope (12).

8. The apparatus of claim 7, **characterized in that** the support (42) for fixing the endoscope (12) supports the endoscope (12) in a predetermined rotary position with respect to a longitudinal axis of the endoscope (12).

9. The apparatus of claim 5 or 8, **characterized in that** the first rail (44) comprises a first positioning scale (46) and the first support (42) comprises a first scale rider (48) that is axially displaceable on the first rail (44) for axial positioning of the support (42).

10. The apparatus of anyone of claims 1 through 9, **characterized in that** the second supporting device (38) comprises a second support (56) that is displaceable on a second rail (54) in longitudinal direction of the rail (54).

11. The apparatus of claim 10, **characterized in that** the second rail (54) comprises a second positioning scale (58) and the second support (56) comprises a scale rider (60) that is displaceable on the second rail (54) for axial positioning the second support (56) on the second rail (54).

12. The apparatus of claim 10 or 11, **characterized in that** the first rail (44) and the second rail (54) are connected with each other via a rotary joint (66).

13. The apparatus of claim 12, **characterized in that** a third positioning scale (68) for adjusting the angle between the first rail (44) and the second rail (54) is circumferentially arranged at the rotary joint (66).

14. The apparatus of claim 13, **characterized in that** a set disk is arranged at the rotary joint (66), the set disk comprising the third positioning scale (68), and which is rotatable relative to the first rail (44) and relative to the second rail (54).

15. The apparatus of anyone of claims 1 through 14, **characterized in that** it further comprises an illumination light test device (40) for testing a light conducting system of the endoscope (12) to be tested.

16. The apparatus of claim 15, **characterized in that** the illumination light test device (40) comprises a photo detector (72) that is movable into a position in front of the light emerging side of the endoscope (12) and that is connected with a displaying device (88) for displaying the light intensity.

17. The apparatus of claim 16, **characterized in that** the displaying device (88) comprises a calibration device (92), e.g. a potentiometer, by means of which the displaying device (88) is adjustable to a reference value.

18. The apparatus of claim 16 or 17, **characterized in that** the illumination light test device (40) comprises a light cable connection (84) for connecting a light cable, wherein the photo detector (72) can be brought into a position in which it is arranged adjacently to the light cable connection (84).

19. The apparatus of anyone of claims 16 through 18, as far as these are referenced back to claim 10 or 11, **characterized in that** the photo detector (72) is fixed at a third supporting device (76), which comprises a support (78) pivotably arranged at an arm (82) that is connected with the second rail (54).

## Revendications

1. Dispositif destiné à tester l'état du système optique d'un endoscope (12), comportant un premier dispositif de maintien (34) pour l'endoscope (12), comportant une image test (36) et un deuxième dispositif de maintien (38) pour l'image test (36), **caractérisé en ce que** l'image test (36) présente, se raccordant à l'intérieur à son secteur extérieur (94), un motif qui présente une ligne de référence (100) circulaire servant de valeur de consigne pour la direction idéale d'observation et l'angle idéal du champ de vision, ainsi qu'un champ de tolérances qui est formé d'anneaux (96, 98) disposés des deux côtés de la ligne de référence (100), de sorte que le motif permet de vérifier la direction d'observation réelle et l'angle réel du champ de vision à l'intérieur de valeurs de tolérance, prédéterminées par le champ de tolérances, de la valeur de consigne pour la direction d'observation et l'angle de champ visuel.

2. Dispositif selon la revendication 1, **caractérisé en ce que** l'image test présente un motif qui permet de vérifier la qualité de l'image.

3. Dispositif selon la revendication 1 ou 2, **caractérisé en ce que** le premier dispositif de maintien (34) et le deuxième dispositif de maintien (38) peuvent être positionnés l'un par rapport à l'autre, de manière que la position relative de l'image test (36) par rapport à l'endoscope (12) soit réglable de manière définie et spécifique à l'endoscope.

4. Dispositif selon la revendication 3, **caractérisé en ce que** la distance entre l'image test (36) et l'endoscope (12) est réglable.

5. Dispositif selon la revendication 3 ou 4, **caractérisé en ce qu'**un angle entre une normale à l'image test (36) et la direction longitudinale de l'endoscope (12) est réglable.

6. Dispositif selon l'une des revendications 3 à 5, **caractérisé en ce qu'**à des données caractéristiques spécifiques de l'endoscope (12) sont associées des valeurs de position pour positionner l'image test (36) et/ou l'endoscope (12).

7. Dispositif selon l'une des revendications 1 à 6, **caractérisé en ce que** le premier dispositif de maintien (34) comporte au moins un premier support (42) qui peut coulisser sur un premier rail (44), dans la direction longitudinale de ce rail (44), lequel s'étend dans la direction longitudinale de l'endoscope (12).

8. Dispositif selon la revendication 7, **caractérisé en ce que** pour fixer l'endoscope (12), le support (42) maintient l'endoscope (12) dans une position angulaire prédéterminée par rapport à une axe longitudinal de l'endoscope (12).

9. Dispositif selon la revendication 5 ou 8, **caractérisé en ce que** le premier rail (44) comporte une première échelle de positionnement (46) et le premier support (42) comporte un premier curseur d'échelle (48) qui peut coulisser axialement sur le premier rail (44), en vue du positionnement axial du support (42).

10. Dispositif selon l'une des revendications 1 à 9, **caractérisé en ce que** le deuxième dispositif de maintien (38) comporte un deuxième support (56) qui peut coulisser sur un deuxième rail (54), dans la direction longitudinale de ce rail (54).

11. Dispositif selon la revendication 10, **caractérisé en ce que** le deuxième rail (54) comporte une deuxième échelle de positionnement (58) et le deuxième support (56) comporte un curseur d'échelle (60) qui peut coulisser axialement sur le deuxième rail (54), en vue du positionnement axial du deuxième support (56) sur le deuxième rail (54).

12. Dispositif selon l'une des revendications 10 ou 11, **caractérisé en ce que** le premier rail (44) et le deuxième rail (54) sont reliés entre eux par une articulation tournante (66).

13. Dispositif selon la revendication 12, **caractérisé en ce que** sur l'articulation tournante (66) est disposée une troisième échelle de positionnement (68), disposée périphériquement, pour le réglage de l'angle entre le premier rail (44) et le deuxième rail (54).

14. Dispositif selon la revendication 13, **caractérisé en ce que** sur l'articulation tournante (66) est disposé un disque de réglage qui comporte la troisième échelle de positionnement (68) et qui peut tourner par rapport au premier rail (44) et par rapport au deuxième rail (54).

15. Dispositif selon l'une des revendications 1 à 14, **caractérisé en ce qu'**il comporte en outre un dispositif de test optique (40) pour tester un système de guidage optique de l'endoscope (12) à tester.

16. Dispositif selon la revendication 15, **caractérisé en ce que** le dispositif de test optique (40) comporte un photodétecteur (72) qui est déplaçable devant l'extrémité côté sortie de lumière de l'endoscope (12), et qui est relié à un dispositif d'affichage (88) pour l'affichage de l'intensité lumineuse.

17. Dispositif selon la revendication 16, **caractérisé en ce que** le dispositif d'affichage (88) comporte un dispositif de calibrage (92), par exemple un potentiomètre, au moyen duquel le dispositif d'affichage (88) est réglable sur une valeur de référence.

18. Dispositif selon la revendication 16 ou 17, **caractérisé en ce que** le dispositif de test optique (40) comporte un branchement de câble optique (84) pour le branchement d'un câble optique, le photodétecteur (72) pouvant être amené dans une position dans laquelle il est disposé au voisinage du branchement de câble optique (84).

19. Dispositif selon l'une des revendications 16 à 18, dans la mesure où elles se rattachent à la revendication 10 ou 11, **caractérisé en ce que** le photodétecteur (72) est fixé à un troisième dispositif de maintien (76) qui comporte un support (78) monté pivotant sur un bras (82) relié au deuxième rail (54).
